# EUROPEAN PATENT APPLICATION

(11) **EP 2 145 621 A1**
(43) Date of publication of application: **20.01.2010**
(21) Application number: 08160550.3
(22) Date of filing: 16.07.2008
(51) Int. Cl.: A61K 31/47, A61P 13/00

(54) **Prevention of dehydration and reduction of gluconeogenetic losses of amino acids by means of propane-1,2,3-triol**

(71) Applicant: Lück, Stephan, 50823 Köln (DE)
(72) Inventor: Lück, Stephan, 50823 Köln (DE)
(74) Representative: Helbing, Jörg

(57) **Abstract**

This invention relates to use of glycerol-containing solutions in geriatric care for reduction of urinary levels and /or for supporting fasting individuals.

## Description

The present invention provides use of glycerol containing preparations for reducing urinary volumes in individuals reluctant to consume liquid. The invention further provides glycerol containing preparations that diminish loss of protein during short- and long-duration fasting.

### Background of the Invention

Dehydration is a common problem in geriatric care as well as in fasting.

On the one hand, elderly people, who per se display reduced fluid intakes, tend to abstain from fluid consumption in the late afternoon and evening hours in order to avoid frequent urination overnight. A comparable behaviour may be seen generally in humans facing situations where urination is impossible or inconvenient (e.g. long-distance car driving): Frequently, such situations are associated with short duration fasting. The combination of dehydration and hypoglycaemia that may result is undesirable and may even be hazardous.

On the other hand, dehydration is also relevant for people undergoing deliberate fasting. Reducing food intake also reduces water intake. In consequence, fasting subjects practically never increase their fluid intake spontaneously and sufficiently to compensate for these losses. Nutritional consultants therefore prescribe strict schemes of fluid intake for fasting subjects. However, this still leaves various problems. In particular during the early stages of fasting, subjects rely on glucose production through gluconeogenesis. During this process, the natural metabolite glycerol (propane-1,2,3-triol), which is a component of triacylglycerols, is liberated in the process of lipolysis and easily converted to glucose in the liver. Nevertheless, its amount is by far not sufficient to ensure glucose production in the order of 100 g to 160 g per day. As a consequence, amino acids become substrates of gluconeogenesis, resulting in undesirable consumption of protein. This ensuing protein loss is aggravated in physically active subjects because, in contrast to non-fasting subjects, protein is utilized as energy source for physical activity.

As stated supra, glycerol is a natural metabolite and non-toxic in large doses - European food laws permit application quantum satis. When consumed, it is rapidly absorbed from the gastrointestinal tract. Besides serving as substrate for gluconeogenesis, glycerol can be oxidized in many tissues (via glycerol dehydrogenase reaction) or be used for re-esterification of fatty acids [van Hall G, Sacchetti M, Radegran G, Saltin B: Human skeletal muscle fatty acid and glycerol metabolism during rest, exercise and recovery. J Physio. 543: 1047-1058, 2002]. This, however occurs only at a slow rate, so that the disappearance rate of plasma glycerol is much slower than that of, for example, glucose.

Distribution space of glycerol depends on plasma concentrations: in the range of low plasma concentrations (< 0.5 mmol/l) it approximates 0.3 l per kg body weight. With larger plasma concentrations, distribution space increases to about 0.6 l/kg, i.e. near to total body water volume [Beylot M, Martin C, Beaufrere B, Riou J P, Mornex R: Determination of steady state and nonsteady-state glycerol kinetics in humans using deuterium-labeled tracer. J Lipid Res, 28: 414-422, 1987; Wade A J.: The distribution and metabolism of glycerol in the rabbit. Bio-chem J, 196(2): 547-556, 1981]. Below plasma concentrations of 1 mmol/l, glycerol is fully reabsorbed by the kidneys. Above this concentration, tubular reabsorption and renal excretion augment over a large range of concentrations [Swanson R E, Thompson R B: Renal tubular handling of glycerol and ethylene glycol in the dog, Am J Physiol 217: 553-562, 1969].

WO 94/25031 discloses an exercise regimen which enhances exercise endurance and performance. The regimen includes pre-exercise hydration with a glycerol solution combined with hydration during exercise with a glycerol based solution to prolong hydration effects. The first pre-exercise glycerol solution regimen begins 2 h prior to exercise and ends ½ h before exercise begins. The hydration during exercise regimen combines glycerol with a carbohydrate and sodium to prolong fluid retention.

Hence, the problem underlying present invention is the need for reducing dehydration and ullage in different person groups that is caused by the reluctance to consume an appropriate amount of liquid. The present invention presents glycerol as suitable agent that fulfils this task.

### Short Description of the Invention

It was found that glycerol can be used for production of preparations that can be applied for reduction of dehydration, e.g. in individuals that are prone to avoid urination like the elderly or individuals that undergo short- or long-duration fasting. In addition, these preparations compensate for loss of amino acids resulting from use of protein as energy source during fasting in conjunction with additional physical activity. More preferably, said preparations are liquid compositions, and even more preferably, said liquid compositions are beverages containing glycerol. In a further preferred embodiment, the glycerol concentration in the composition is from 0.2 to 1.0 mol/l. In a further embodiment, preparation comprises additional compounds improving palatability and/or further nutritional supplements such as electrolytes, vitamins, vitamin-related additives, proteins, amino acids, dyes, flavouring agents, stabilizing agents, and functional additives. Thus, the present invention provides:
(1) the use of liquids containing glycerol for application in geriatric care, for reduction of the need to urinate, and for supporting fasting subjects;
(2) a preferred embodiment of aspect (1), wherein the liquid is a beverage containing glycerol in the range of 0.2 to 1.0 mol/l;
(3) the liquid composition of aspect (1), wherein the preparation is a beverage containing glycerol in the range of 0.2 to 1.0 mol/l, preferably 0.29 to 1.0 mol/l;
(4) a preferred embodiment of aspect (3), wherein the preparation is a beverage containing glycerol in the range of 0.2 to 1.0 mol/l.

### Short Description of the Figures

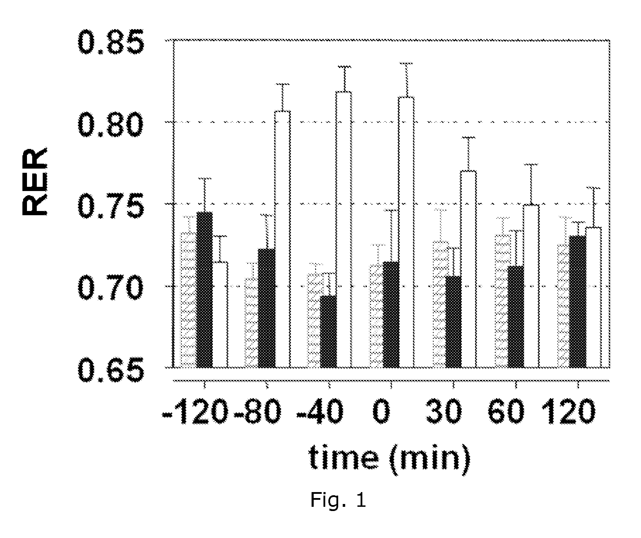

Fig. 1: Respiratory exchange ratio (RER, means ± SE) in 8 fasting male subjects after administration of isoosmotic glycerol solution (290 mmol/l, hatched bars), hyperosmotic glycerol solution (580 mmol/l, filled bars) and carbohydrate solution (60 g/l, white bars). 2 l of each beverage were consumed between -120 min and -40 min.

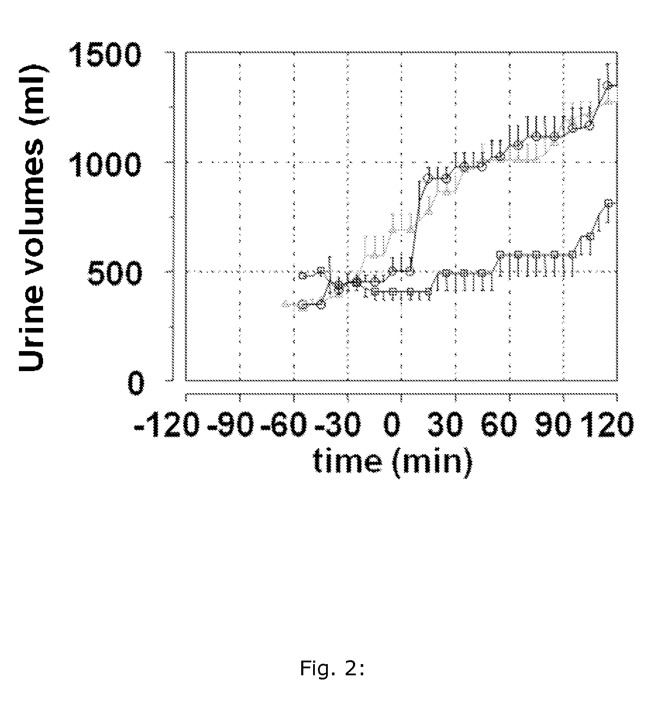

Fig. 2: Cumulated urine volumes (means ± SE) in 8 fasting male subjects after administration of isoosmotic glycerol solution (290 mmol/l, triangles), hyperosmotic glycerol solution (580 mmol/l, squares) and carbohydrate solution (60 g/l). 2 l of each beverage were consumed between -120 min and -40 min.

### Detailed Description of the Invention

The present invention describes use of glycerol in liquid compositions. Glycerol is membrane-permeable and is distributed equally on membrane tissue. Due to the resulting osmotic effect, an intracellular influx of water is observed upon application of a glycerol-containing solution of a concentration above that of an isoosmotic glycerol solution (∼ 290 mmol/l). Therefore, consuming a composition of the present invention indicates rather a lack than an overload of water from the viewpoint of osmolarity. As a consequence, the intravascular space is not overloaded with water, resulting in a reduction of urine (hormonal regulation).

In one aspect, the compositions of the present invention serve as a means for reducing dehydration in individuals that are reluctant to consume sufficient amount of water. In a general embodiment, this invention can benefit any individual being forced to undergo a longer period in which urination is impossible or inconvenient, e.g. long-distance car driving etc. In a preferred embodiment, this invention is specifically valuable to older people who tend to consume too little water in the evenings so not to be forced to get up at night in order to urinate. This behaviour deteriorates the effect of already diminished thirst sensation among the elderly. Therefore, in one aspect, this invention is specifically targeted at improving living conditions of the elderly, but also to psychologically help persons forced to undergo situation where urination is impossible.

In a further aspect, the present invention supports people deliberately undergoing a short- or long-duration fasting period. In analogy to what is stated supra, the present invention reduces water loss and thereby diminishes ullage. This is especially critical in fasting individuals, who are also prone to consume too little water. This invention can therefore help them to further substantiate their fluid intake. In summary, as one aspect of the present invention, the surprising advantage is that a liquid composition comprising glycerol may lead to long-lasting hydration with low urine volumes (Fig. 1).

In a further aspect of the present invention, it was found that another negative effect in fasting individuals can be alleviated by use of a composition of the present invention. Specifically, gluconeogenesis is the main source of glucose during the first stages of fasting. By said process, glycerol can be transformed into glucose, yet the body's own glycerol reservoir is by far not sufficient to ensure adequate glucose production. In consequence, the body resorts to amino acids as substrates for gluconeogenesis, resulting in undesired protein loss. Physical activity exacerbates this loss of protein. Glycerol supplied by the compositions of present invention can be used by the organism as an energy source. However, as a surprising effect, glycerol does not affect blood glucose level and does not increase respiratory exchange ratio, which means it does not increase the contribution of carbohydrates to energy supply (Fig. 2). In post-absorptive states, increased glycerol availability enhances glycerol-based gluconeogenesis and thus reduces conversion of amino acids to glucose.

Therefore, in a further aspect, solutions of the present invention contain at least 0.2 to 1.0 mmol/l, preferably 0.29 to 1.0 mmol/I glycerol, more preferably 0.29 to 0.6 mmol/l.

Of note, solutions containing too high concentrations of glycerol can potentially lead to stomach upsets, vomiting and diarrhoea in healthy subjects. Similarly, fast consumption of large doses may lead to cerebral dehydration resulting in headaches, dizziness and blurred vision.

Similarly, application of glycerol may cause hypervolemia and is contraindicated in subjects with reduced heart function or hepatic or renal diseases or when reduction of urine flow is not desired.

Therefore, in another aspect of present invention, compositions do not contain more than 1 mol/I of glycerol.

Additionally, in a further aspect, the composition contains additives such as electrolytes, vitamins, vitamin-related additives, amino acids, proteins, dyes, flavouring agents, and stabilizing agents.

Added electrolytes are chosen from the group of sodium, potassium, calcium, chloride, phosphorous, and magnesium. Too high concentrations of electrolytes could have negative effects on electrolyte resorption and general physical performance, wherefore the concentration range is chosen so that positive and no negative effects are obtained. Therefore, in a further aspect of the present invention, the concentration of electrolytes in the composition is:
10 - 400 mg/l sodium, preferably about 300 mg/l; and/or
10 - 300 mg/l potassium, preferably about 200 mg/l; and/or
10 - 200 mg/l chloride, preferably about 150 mg/l; and/or
10 - 200 mg/l phosphorous, preferably about 150 mg/l; and/or
5 - 200 mg/l magnesium, preferably about 100 mg/l; and/or
5 - 200 mg/l calcium, preferably about 100 mg/l.

In a further aspect, vitamins and/or vitamin-related additives are added to the solution. In a preferred embodiment, the vitamins and/or vitamin-related additives added are chosen from vitamin B1, vitamin B11, vitamin B12, vitamin C, and L-carnitine. More preferably, the preferred concentrations of the vitamins and vitamin-related additives added depend upon an undersupply or specific demandand are:
1 - 100 mg/l ascorbic acid (vitamin C), preferably about 30 mg/l; and/or
0.50 - 5 mg/l thiamine (vitamin B1), preferably about 1.0 mg/l; and/or
100 - 600 µg/l folic acid/folat/pteroylglutamate (vitamin B11), preferably about 400 µg/l; and/or
1-10 µg/l cobalamine (vitamin B12), preferably about 5 µg; and/or
100 - 4000 mg L-carnitine, preferably about 1000 mg.

In a further aspect, flavouring agents and dyes are added to confer an appealing appearance to the composition. Suitable flavouring agents and dyes are chosen from all authorized additives, especially natural flavouring agents and dyes (phenolic compounds and isoprenoidic compounds like phenolics, carotenoids, flavonoids, terpenes etc.)

In a further aspect, stabilizing agents are contained in the composition. In a more preferred embodiment, stabilizing agents are chosen from the group of benzoic acid, propionic acid, and antioxidants.

In a further embodiment, the present invention comprises an instant composition, from which the liquid composition of the present invention can be generated by dissolution in water. This instant composition can be on hand as a powder or as syrupy liquid or paste.

The following examples will further expatiate on the benefits of present invention. These examples however are not to be construed as limiting the invention.

### Examples

Example 1: Determination of the respiratory exchange rate upon administration of carbohydrate, isoosmotic, and hyperosmotic glycerol solution.

The respiratory exchange ratio (RER) upon administration of carbohydrate (60 g/l), isoosmotic (290 mmol/l), and hyperosmotic (580 mmol/l) glycerol solution was studied in 8 male subjects aged between 22 and 35 years. After an overnight fast, each subject consumed 2 l of one solution (between - 120 min and -40 min in Fig.1). All subjects had to ingest all types of solution, however in random order. Oxygen consumption and CO₂ output for RER determination were measured over 5 min periods at the times given in Fig. 1. Simultaneously, blood samples were taken and analyzed for plasma glucose and haemoglobin concentration as well as haematocrit. RER measurements confirmed that all subjects were in a fasting state. In contrast to the carbohydrate solution, RER remained in the fasting range after administration of the glycerol beverages. As expected, plasma glucose concentration increased after carbohydrate ingestion and glucose served as energy source as indicated by the increase in RER. This shows that consumption of glycerol does not affect the energy metabolism during fasting.

Example 2: Determination of the cumulated urine volumes upon administration of carbohydrate, isoosmotic, and hyperosmotic glycerol solution.

The cumulated urine volumes upon administration of carbohydrate (60 g/l), isoosmotic (290 mmol/l), and hyperosmotic (580 mmol/l) glycerol solution was studied in 8 male subjects aged between 22 and 35 years. After an overnight fast and emptying their urinary bladder, each subject consumed 2 l of one solution (between - 120 min and -40 min in Fig. 2). All subjects had to ingest all types of solution, however in random order. Urine was collected over a period of 3 hours (between - 60 min and 120 min in Fig. 2). While the isoosmotic glycerol solution induced only a short delay of urine output in comparison with the carbohydrate beverage, the hyperosmotic glycerol concentration lead to a substantial reduction of urine production over the measurement period.

## Claims

1. Use of liquids containing glycerol for application in geriatric care, for reduction of the need to urinate, and/or for supporting fasting subjects.

2. The use of claim 1, wherein the liquid is a beverage containing glycerol in the range of 0.2 to 1.0 mol/l, preferably in the range 0.29 to 1.0 mol/l.

3. The use of claim 1 or 2, wherein the preparation comprises further compounds improving palatability and/or further nutritional supplements such as electrolytes, vitamins, vitamin-related additives, proteins, amino acids, dyes, flavouring agents and stabilizing agents

4. The use of claim 3, wherein electrolytes are chosen from sodium, potassium, magnesium, calcium, chloride, and phosphorous.

5. The use of claim 3 or 4 wherein the content of mineral nutrients per liter is
10 - 400 mg sodium, preferably about 300 mg; and/or
10 - 300 mg potassium, preferably about 200 mg; and/or
10 - 200 mg chloride, preferably about 150 mg; and/or
10 - 200 mg phosphorous, preferably about 150 mg; and/or
5 - 200 mg magnesium, preferably about 100 mg; and/or
5 - 200 mg calcium, preferably about 100 mg.

6. The use of any one of claims 3 to 5, wherein
(i) vitamins and vitamin-related additives are chosen from vitamin B1, vitamin B11, vitamin B12, vitamin C, and L-carnitine and wherein preferably the content of vitamins and vitamin-related additives per liter is 1 - 100 mg vitamin C, preferably about 30 mg; and/ or 0.50 - 5 mg vitamin B1, preferably about 1.0 mg; and/or 100 - 600 µg vitamin B11, preferably about 400 µg; and/or 1 - 10 µg vitamin B12, preferably about 5 µg; and/or 100 - 4000 mg L-carnitine, preferably about 1000 mg; and/or
(ii) the flavouring agent is chosen from all authorized additives, especially natural flavouring agents and dyes, more preferably phenolic compounds and isoprenoidic compounds like phenolics, carotenoids, flavonoids, terpenes; and/or
(iii) the stabilizing agent is chosen from benzoic acid, propionic acid, and antioxidants.

7. The use of any one of claims 1 to 6, wherein preparation is obtained from a liquid or solid concentrate.

8. A liquid composition containing glycerol for treatment for reduction of the need to urinate, in geriatric care, and/or for supporting fasting subjects.

9. The liquid composition of claim 8, wherein the preparation is a beverage containing glycerol from 0.2 to 1.0 mol/l, preferably from 0.29 to 1.0 mol/l.

10. The liquid composition of claim 8 or 9, wherein the preparation comprises further compounds improving palatability and/or further nutritional supplements such as electrolytes, vitamins, vitamin-related additives, proteins, dyes, amino acids, flavouring agents and stabilizing agents

11. The liquid composition of claim 10, wherein electrolytes are chosen from sodium, potassium, magnesium and calcium, chloride and phosphorous.

12. The liquid composition of claim 10 or 11, wherein the content of electrolytes per liter is
10 - 400 mg sodium, preferably about 300 mg; and/or
10 - 300 mg potassium, preferably about 200 mg; and/or
10 - 200 mg chloride, preferably about 150 mg; and/or
10 - 200 mg phosphorous, preferably about 150 mg; and/or
5 - 200 mg magnesium, preferably about 100 mg; and/or
5 - 200 mg calcium, preferably about 100 mg.

13. The liquid composition of any one of claims 10 to 12, wherein
(i) vitamins and vitamin-related additives are chosen from the group of vitamin B1, vitamin B11, vitamin B12, vitamin C, and L-carnitine and wherein preferably the content of vitamins and vitamin-related additives per liter is 1 - 100 mg vitamin C, more preferably about 30 mg; and/or 0.50 - 5 mg vitamin B1, more preferably about 1.0 mg; and/or 100 - 600 µg vitamin B11, more preferably about 400 µg; and/or 1-10 µg vitamin B12, more preferably about 5 µg; and/or 100 - 4000 mg L-carnitine, more preferably about 1000 mg; and/or
(ii) the flavouring agent is chosen from all authorized additives, especially natural flavouring agents and dyes, more preferably phenolic compounds and isoprenoidic compounds like phenolics, carotenoids, flavonoids, terpenes; and/or
(iii) the stabilizing agent is chosen from the group of benzoic acid, propionic acid, and antioxidants.

14. The liquid composition of any one of claims 8 to 13, wherein the liquid composition is obtained from a liquid or solid concentrate.
